(19) Europäisches Patentamt
European Patent Office
Office européen des brevets

(11) **EP 2 936 091 B1**

(12) **EUROPÄISCHE PATENTSCHRIFT**

(45) Veröffentlichungstag und Bekanntmachung des Hinweises auf die Patenterteilung:
**10.04.2019 Patentblatt 2019/15**

(21) Anmeldenummer: **13789501.7**

(22) Anmeldetag: **22.10.2013**

(51) Int Cl.:
*G01J 1/44* (2006.01)          *G01J 3/28* (2006.01)
*G01J 3/44* (2006.01)          *A61B 5/1455* (2006.01)

(86) Internationale Anmeldenummer:
**PCT/EP2013/072088**

(87) Internationale Veröffentlichungsnummer:
**WO 2014/095128 (26.06.2014 Gazette 2014/26)**

(54) **VORRICHTUNG UND VERFAHREN ZUR MESSUNG EINES PERIODISCHEN SIGNALS**

APPARATUS AND METHOD FOR MEASURING A PERIODIC SIGNAL

DISPOSITIF ET PROCÉDÉ DE MESURE D'UN SIGNAL PÉRIODIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2012 DE 102012113008**

(43) Veröffentlichungstag der Anmeldung:
**28.10.2015 Patentblatt 2015/44**

(73) Patentinhaber: **Hamilton Bonaduz AG
7402 Bonaduz (CH)**

(72) Erfinder:
• **OFFENBECK, Bernd
93057 Regensburg (DE)**
• **ARQUINT, Philipp
CH-7402 Bonaduz (CH)**

(74) Vertreter: **Caspary, Karsten et al
Kroher-Strobel
Rechts- und Patentanwälte PartmbB
Bavariaring 20
80336 München (DE)**

(56) Entgegenhaltungen:
**WO-A1-01/78593      US-A1- 2002 012 152**

• **Mark Looney: "Advanced Digital Post-Processing Techniques Enhance Performance in Time-Interleaved ADC Systems", Analog dialogue 37-8, August 2003, 31. August 2003 (2003-08-31), Seiten 1-5, XP055099544, Gefunden im Internet: URL:http://www.analog.com/library/analogdialogue/archives/37-08/post_processing.pdf [gefunden am 2014-01-30]**

EP 2 936 091 B1

**Beschreibung**

Die vorliegende Erfindung betrifft das Gebiet der Prozessmesstechnik und -analytik und insbesondere eine Vorrichtung und ein Verfahren zur Messung eines periodischen Signals.

In Sensoren der Prozessmesstechnik bzw. -analytik werden immer häufiger optische Messverfahren eingesetzt, bei welchen sich die Amplitude und/oder die Phasenlage eines optischen Signals nach Auftreffen auf ein Messobjekt ändern und das veränderte Signal von einem optischen Sensor erfasst wird. Ein Anwendungsbeispiel für ein derartiges optisches Messverfahren ist die Messung des Sauerstoffgehalts bzw. der Sauerstoffsättigung einer Flüssigkeit oder eines Stoffs, wobei zum Beispiel ein Farbstoff mittels eines Lichtsignals einer vorbestimmten Wellenlänge, Amplitude und Phasenlage beleuchtet wird und das vom Farbstoff reflektierte Lumineszenzlicht analysiert wird. Ändert sich die Sauerstoffkonzentration in dem Farbstoff, dann ändert sich auch die Abklingzeit der Lumineszenz und somit die Amplitude und Phasenlage des empfangenen optischen Signals. Bei entsprechender Kalibrierung sind damit Amplitude und Phase des empfangenen optischen Signals ein Maß für die Sauerstoffkonzentration.

Im Stand der Technik werden zur Erfassung der optischen Signale in Sensoren der Prozessmesstechnik bzw. -analytik heute im Wesentlichen zwei Verfahren verwendet.

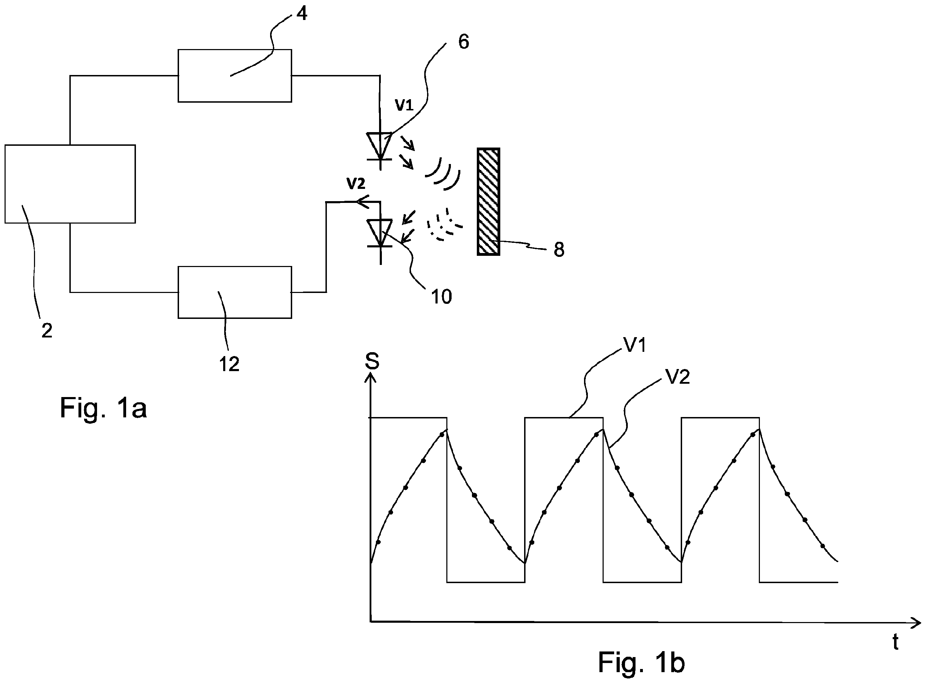

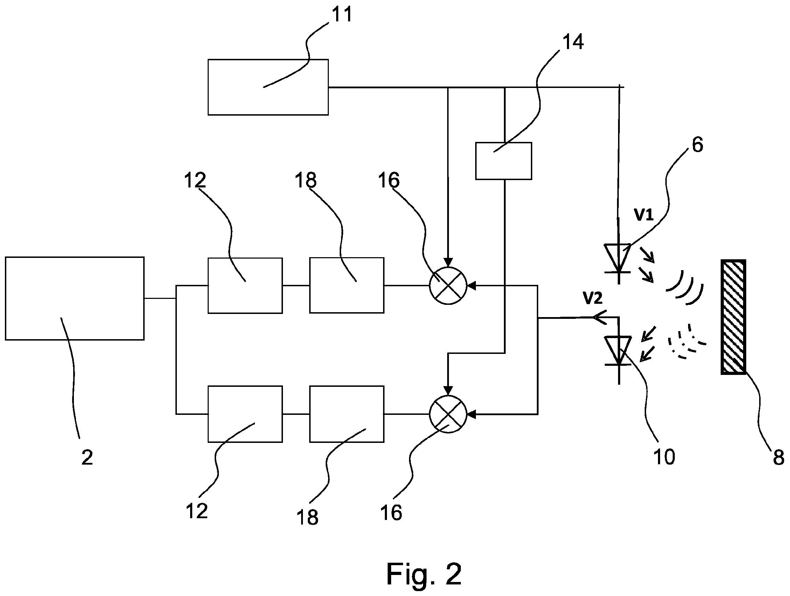

Beim ersten Verfahren, dessen Vorrichtung bzw. Signalverlauf schematisch in den Fig. 1a und 1b abgebildet sind, wird zunächst gesteuert von einer Steuereinrichtung 2 ein elektrisches Signal mittels eines Digital-Analog-Wandlers (DAC) 4 erzeugt und von einer LED 6 in ein optisches Signal V1 umgewandelt. Das von einem Farbstoff 8 reflektierte optische Signal wird in einer Photodiode 10 in ein elektrisches Messsignal V2 umgewandelt und anschließend vollständig mit einem Analog-Digital-Wandler (ADC) 12 erfasst und digital in der Steuereinrichtung 2 verarbeitet und ausgewertet. Damit eine möglichst genaue Erfassung des elektrischen Messsignals V2 im ADC 12 erfolgen kann, ist dort eine hohe Überabtastung erforderlich. Die Abtastpunkte sind in Fig. 2 schematisch als kleine Vollkreise dargestellt. Zum Beispiel ist bei einem Signal der Frequenz 8 kHz eine Abtastung mit typischerweise 80 bis 800 kSPS notwendig. Derartige Analog-Digital-Wandler mit einer hohen Überabtastung weisen eine komplexe digitale Logik auf, und deshalb ist ihr Energieverbrauch relativ hoch. Darüber hinaus fällt bei der hohen Überabtastung eine große Menge an digitalen Daten an, die von einem leistungsfähigen Prozessor weiter verarbeitet werden müssen.

Bei einem zweiten bekannten Messverfahren, für das in Fig. 2 beispielhaft ein Sensor schematisch dargestellt ist, wird ein elektrisches Messsignal in einem Signalerzeuger 11 erzeugt, wie beim ersten bekannten Verfahren von einer LED 6 in ein optisches Signal V1 gewandelt, das optische Signal V1 auf einen Farbstoff 8 gerichtet, das von dem Farbstoff reflektierte optische Messsignal in der Photodiode 10 in das elektrische Messsignal V2 umgewandelt und dieses mittels eines analogen Lock-in-Verstärkers verarbeitet. Der Lock-In-Verstärker umfasst einen Phasenverschieber 14 und zwei analoge Mischer bzw. Multiplizierer 16. Die von einem Tiefpass 18 gefilterten niederfrequenten Anteile des Messsignals werden von einem Analog-Digital-Wandler (ADC) 12 erfasst und in einer Steuereinrichtung 2 ausgewertet.

Nachteilig an diesem zweiten Verfahren ist, dass die Eigenschaften des analogen Mischers stark durch Bauteildriften beeinflusst werden. Bei Sensoren mit eingebauter Elektronik, die in der Prozessmesstechnik in einem erweiterten Temperaturbereich eingesetzt werden und lange Betriebszeiten ohne Neukalibrierung überstehen müssen, ist ein derartiges Bauteildriften jedoch nicht akzeptabel. Insbesondere ist der Einsatz von derartigen Sensoren in Zonen mit erhöhter Explosionsgefahr aufgrund der hohen Energieaufnahme nicht möglich.

Das US-Patent 8,078,246 offenbart einen Sensor für die Pulsoximetrie, bei dem das von einer Photodiode umgewandelte elektrische Messsignal einem Eingangsverstärker zugeführt wird und anschließend auf eine Anzahl von N Messkanälen verteilt wird, die jeweils unterschiedliche Wellenlängen des verstärkten Messsignals verarbeiten. Jeder Messkanal umfasst dabei einen analogen Schalter, einen Tiefpassfilter sowie einen Analog-Digital-Wandler, und die Ausgangssignale der N Messkanäle werden in einer Steuereinrichtung ausgewertet. Auch hier gilt das in Bezug auf das erste bekannte Verfahren genannte Problem, dass die Signalauswertung pro Messkanal nur dann auch genau ist, wenn der Analog-Digital-Wandler eine ausreichend hohe Auflösung und damit eine entsprechend hohe Überabtastung aufweist. Die oben genannten Schwierigkeiten treten deshalb auch im Zusammenhang mit der US 8,078,246 auf.

Es ist daher die Aufgabe der vorliegenden Erfindung, eine Vorrichtung und ein Verfahren zur Messung eines periodischen Signals bereitzustellen, die bzw. das die Nachteile des Standes der Technik überwindet, eine relativ geringe Leistungsaufnahme und damit Eigenerwärmung aufweist, einfache und günstige Bauelemente enthält und eine effiziente und genaue Messung gewährleistet.

Diese Aufgabe wird durch den Gegenstand der unabhängigen Ansprüche 1 und 8 gelöst. Vorteilhafte Ausführungsformen sind Gegenstand der abhängigen Ansprüche.

Erfindungsgemäß wird eine Vorrichtung zur Messung eines periodischen Signals mit einer ersten Steuereinrichtung zur Erzeugung eines periodischen elektrischen Eingangssignals der Periode T, einer Lichtquelle zur Erzeugung eines auf ein Messobjekt gerichteten optischen Eingangssignals aus dem elektrischen Eingangssignal der Periode T, einem optischen Empfänger zur Erfassung und Umwandlung des von dem Messobjekt reflektierten Signals, das dem in Phase und Amplitude veränderten optischen Eingangssignal entspricht, in ein elektrisches Messsignal der Periode T, und einer Mehrzahl von parallel zwischen dem optischen Empfänger und einer zweiten Steuereinrichtung gestalteten Messkanälen bereitgestellt, die jeweils in Reihe geschaltet ein Schaltelement, ein Filterelement und einen Analog-Digital-Wandler aufweisen, wobei die zweite Steuereinrichtung zur Auswertung der Messsignale von der Mehrzahl der Messkanäle geeignet ist. Die erfindungsgemäße Vorrichtung zeichnet sich dadurch aus, dass an der Mehrzahl von Messkanälen jeweils das elektrische Messsignal anliegt, die erste Steuereinrichtung mit der Mehrzahl von Schaltelementen verbunden und dazu eingerichtet ist, die Schaltelemente für jeweils unterschiedliche Zeitintervalle derart anzuschalten, dass das elektrische Messsignal wiederholt in gleich langen, pro Messkanal disjunkten Zeitabschnitten abgegriffen wird, so dass sich die Zeitabschnitte in jedem der Messkanäle nicht überlappen und die Analog-Digital-Wandler eine maximale Abtastrate kleiner als $2 \times \dfrac{1}{T}$ aufweisen.

Durch das "Aufteilen" des elektrischen Messsignals in die Mehrzahl von Messkanälen können die entsprechenden einzelnen Messsignale von langsamen, sparsamen Analog-Digital-Wandlern in Unterabtastung, d.h. unterhalb der Nyquist-Shannon-Abtastrate, erfasst werden. Gleichzeitig können die in den unterschiedlichen Zeitintervallen erfassten Messsignale in der zweiten Steuereinrichtung zusammengefasst und damit das gesamte Messsignal exakt erfasst und verarbeitet werden. Im Gegensatz zu den Vorrichtungen und Verfahren des Standes der Technik ist die Leistungsaufnahme erheblich geringer als bei einer Überabtastung in einem einzigen oder auch mehreren Analog-Digital-Wandlern, auch weil die Schaltelemente eine sehr geringe Leistungsaufnahme aufweisen. Durch die geringere Leistungsaufnahme ergibt sich eine geringere Eigenerwärmung der gesamten Messvorrichtung, und damit ist eine höhere Integrationsdichte der Bauelemente möglich. Zudem ist keine Einlaufdrift durch die Eigenerwärmung zu erwarten, so dass die Vorrichtung sofort einsatzbereit ist. Darüber hinaus ermöglicht die geringe Leistungsaufnahme den Einsatz in explosionsgefährdeten Umgebungen.

Mit besonderem Vorteil ist das elektrische Eingangssignal als Rechtecksignal ausgebildet. Dadurch weist das empfangene Lumineszenzsignal im Wesentlichen eine Sägezahnform auf, die durch die Mehrzahl von Messkanälen der erfindungsgemäßen Vorrichtung besonders einfach und genau verarbeitet werden kann.

Mit weiterem Vorteil ist das Filterelement als Tiefpass ausgebildet. Tiefpässe können beispielsweise aus passiven analogen Bauelementen aufgebaut sein, die günstig und einfach verfügbar sind. Durch die Tiefpassfilterung wird eine Integration des Messsignals in dem jeweiligen Messkanal vorgenommen, d. h. es findet eine Mittelwertbildung statt.

Bevorzugt sind die Schaltelemente in jedem der Mehrzahl von Messkanälen als CMOS-Schalter bzw. -Umschalter ausgebildet. Derartige Schalter in CMOS-Technik sind zuverlässig, einfach aufgebaut und preisgünstig verfügbar und damit für die erfindungsgemäße Vorrichtung besonders gut geeignet.

Mit weiterem Vorteil ist die erste Steuereinrichtung mit der zweiten Steuereinrichtung verbunden und synchronisiert. Damit kann die Signalverarbeitung insgesamt verbessert werden, weil insbesondere die genauen Zeitpunkte der Aussendung des periodischen Messsignals und die Zeitpunkte der Schaltung der Schaltelemente direkt verarbeitet werden können.

Besonders bevorzugt ist es, wenn ein integrierter Schaltkreis die erste Steuereinrichtung, die zweite Steuereinrichtung sowie die Mehrzahl der Analog-Digital-Wandler der Mehrzahl von Messkanälen aufweist. Ein derartiger integrierter Schaltkreis kann beispielsweise ein dafür konstruierter Mikrocontroller sein. Aufgrund der nicht zu komplexen Funktionalität der einzelnen Bauelemente ist ein solcher Mikrocontroller relativ einfach aufgebaut und zu akzeptablen Kosten verfügbar.

Mit weiterem Vorteil weist die erste Steuereinrichtung einen Pulsweitenmodulations- (PWM) Generator auf. Damit können durch eine einfache digitale Logik sowohl das Messsignal erzeugt als auch die Abtast- bzw. Schaltelemente angesteuert werden. In dem Mikrocontroller selbst, in den der PWM-Generator integriert sein kann, ist selbst kein Rechenaufwand notwendig, um die entsprechenden Signale zu erzeugen. Der Einsatz eines PWM-Generators ist deshalb ressourcen- und energiesparend.

Weiterhin erfindungsgemäß ist ein Verfahren zur Messung eines periodischen Signals wie im Anspruch 8 definiert.

Die in Bezug auf die oben genannte Vorrichtung erwähnten Vorteile gelten auch für das erfindungsgemäße Verfahren. Im Vergleich zum erwähnten Stand der Technik (erstes Verfahren) kann man sich vorstellen, dass bei dem erfindungsgemäßen Verfahren ebenso viele Messkanäle verwendet werden, wie beim ersten Verfahren des Stands der Technik Stützpunkte innerhalb einer Periode des gesendeten Signals vorhanden sind. Es wird jedoch nicht nur das Signal an einem einzigen Punkt erfasst, sondern derselbe Bereich des elektrischen Messsignals wird über eine hohe Anzahl von Perioden integriert und damit zuverlässig ein Messmittelwert gebildet. Trotz der höheren Anzahl von relativ einfachen und preisgünstigen Bauelementen kann bei dem erfindungsgemäßen Verfahren und der erfindungsgemäßen Vorrichtung auf aufwändige und teure Analog-Digital-Wandler und besonders leistungsfähige Prozessoren verzichtet werden. Dadurch ergibt sich eine Kostenersparnis ohne bei der Messgenauigkeit und Zuverlässigkeit Abstriche machen zu müssen.

Bevorzugt ist es, wenn sich die Zeitintervalle in jedem der Mehrzahl von Messkanälen nicht überlappen. Insbesondere ist es auch von Vorteil, wenn die Zeitintervalle in jedem der Mehrzahl von Messkanälen gleich lang sind. Damit ist die Verarbeitung in jedem der Messkanäle prinzipiell identisch.

Mit besonderem Vorteil entsprechen die Zeitintervalle in jedem der Mehrzahl von Messkanälen deren Periode T des elektrischen Eingangssignals. Damit wird in jedem Messkanal genau derselbe sich wiederholende Zeitabschnitt über die gesamte Messdauer abgetastet, integriert und analog-digital-gewandelt. Durch Zusammensetzen der einzelnen Ausgangssignale der Mehrzahl von Messkanälen in der zweiten Steuereinrichtung lässt sich so die Amplitude und auf die Phasenlage des Messsignals auf einfache Weise bestimmen.

Mit weiterem Vorteil ist das elektrische Eingangssignal als Rechtecksignal ausgebildet, und bevorzugt ist die erste Steuereinrichtung mit der zweiten Steuereinrichtung synchronisiert.

Besonders bevorzugt ist, wenn in jedem Analog-Digital-Wandler der Mehrzahl von Messkanälen die maximale Abtastrate $< 2 \times \dfrac{1}{T}$ ist. Durch diese geringe Abtastrate, die einer Unterabtastung unterhalb der maximalen Abtastrate gemäß Nyquist-Shannon-Abtasttheorem liegt. Dies reduziert die Anforderungen an die Analog-Digital-Wandler erheblich und senkt deren Leistungsaufnahme sowie entsprechend die Erwärmung des Bauelements in Betrieb.

Durch die erfindungsgemäße Vorrichtung bzw. das erfindungsgemäße Verfahren lassen sich die Ressourcen einer entsprechenden elektronischen Schaltung, insbesondere die eines modernen Mikrocontrollers, effizienter nutzen. Da die Energieaufnahme von Analog-Digital-Wandlern (ADC) üblicherweise mit der Auflösung, das heißt der maximalen Abtastrate, steigt, sind bei der vorliegenden Vorrichtung lediglich Analog-Digital-Wandler mit sehr geringer Energieaufnahme erforderlich, ohne dabei auf eine genügend hohe Auflösung des Messsignals zu verzichten.

Die vorliegende Erfindung wird nachfolgend anhand von bevorzugten Ausführungsformen unter Bezugnahme auf die Zeichnungen beschrieben, in denen:

Fig. 1a eine schematische Darstellung einer ersten optischen Messvorrichtung des Standes der Technik ist;

Fig. 1b eine Kurvendarstellung der Messsignale der in Fig. 1a dargestellten optischen Messvorrichtung ist;

Fig. 2 eine schematische Darstellung einer zweiten optischen Messvorrichtung des Standes der Technik ist;

Fig. 3 eine schematische Darstellung einer bevorzugten Ausführungsform einer erfindungsgemäßen Vorrichtung zur Messung eines periodischen Signals ist; und

Fig. 4 eine Kurvendarstellung der Messsignale der in Fig. 3 dargestellten Vorrichtung ist.

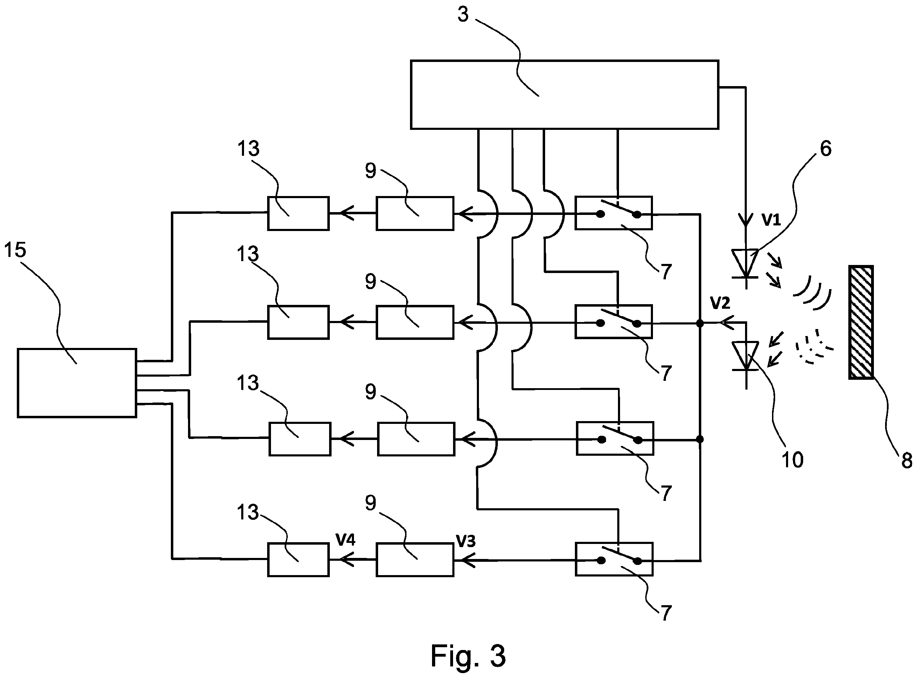

Fig. 3 zeigt eine schematische Darstellung einer bevorzugten Ausführungsform der erfindungsgemäßen Vorrichtung zur Messung eines periodischen Signals. Eine erste Steuereinrichtung 3 erzeugt ein periodisches elektrisches Signal V1 mit einer Periode T, das in einer LED 6 oder einer gleichwertigen Lichtquelle in ein optisches Signal umgewandelt wird. Das optische Signal wird von der LED 6 auf einen Farbstoff 8 gerichtet, wobei anstelle des in der vorliegenden Ausführungsform verwendeten Farbstoffs 8 auch eine anderes Messobjekt verwendet werden kann. Das vom Farbstoff 8 reflektierte Licht wird als optisches Messsignal bezeichnet und auf eine Photodiode 10 gerichtet. Dort wird es in ein elektrisches Messsignal V2 umgewandelt.

Das elektrische Messsignal V2 wird von vier im Wesentlichen gleich aufgebauten Messkanälen abgenommen. Jeder Messkanal weist ein Schaltelement 7, einen Tiefpass 9 und einen Analog-Digital-Wandler (ADC) 13 auf, wobei jedes Schaltelement 7 mit der ersten Steuereinrichtung 2 und der Ausgang jedes ADC 13 mit einer zweiten Steuereinrichtung 15 verbunden ist. Die Anzahl der Messkanäle kann in Abänderung des vorliegenden Ausführungsbeispiels auch kleiner oder größer als vier sein. Dabei gilt: je höher die Anzahl der Messkanäle, desto genauer die Auswertung des elektrischen Messsignals V2.

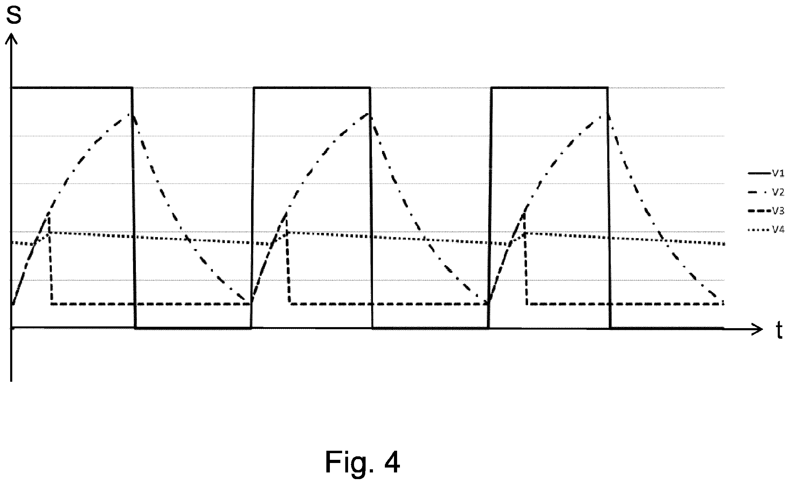

Die Schaltelemente 7 können als Analogschalter oder auch als CMOS-Schalter ausgebildet sein, die beispielsweise von einem PWM-Generator in der ersten Steuereinrichtung 3 geschaltet werden. Dabei wird das elektrische Messsignal V2, das eine Periodendauer aufweist, die der des elektrischen Signals V1 entspricht, wiederholt in gleich langen, disjunkten Zeitabschnitten abgegriffen, und zwar derart, dass derselbe Zeitabschnitt jeder Periode T von demselben Messkanal verarbeitet wird. Eine detaillierte Erläuterung folgt unten unter Bezugnahme auf Fig. 4.

Das Ausgangssignal, im unteren Messkanal in Fig. 3 mit V3 gekennzeichnet, jedes Schaltelements 7, das einen periodisch wiederkehrenden Ausschnitt aus dem elektrischen Messsignal V2 darstellt, wobei sich die Ausschnitte in jedem der Messkanäle nicht überlappen und in der bevorzugten Ausführungsform die Summe aller Ausschnitte pro Messkanal das elektrische Messsignal V2 liefert, wird jeweils einem Tiefpass 9 zugeführt, der die Funktion eines Integrierers bzw. arithmetischen Mittelwertbildners aufweist und die niederfrequenten Anteile des Eingangssignals herausfiltert. Tiefpassfilter können als einfach aufgebaute passive analoge Bauelemente ausgebildet sein, die robust und relativ temperaturunempfindlich sind.

Das Ausgangssignal jedes Tiefpassfilters, im unteren Messkanal in Fig. 3 mit V4 gekennzeichnet, wird einem Analog-Digital-Wandler (ADC) 13 zugeführt. Dort wird es mit einer geringen Abtastrate bzw. Abtastfrequenz, die im bevorzugten Ausführungsbeispiel kleiner als $2 \times \dfrac{1}{T}$ ist, verarbeitet, d.h. digitalisiert. Da die Messsignale in jedem Messkanal nur in einem kleinen Zeitabschnitt der gesamten Periode T vorhanden sind, können vergleichsweise langsame und energetisch sparsame ADCs verwendet werden. Denn in dem Zeitraum, wo kein Signal vorhanden ist, muss nicht abgetastet werden. Dies reduziert die für eine ausreichende Erfassung notwendige Abtastrate erheblich, und damit können sehr viel weniger aufwändige und komplexe ADCs eingesetzt werden, die eine wesentlich reduzierte Leistungsaufnahme und Wärmeentwicklung aufweisen.

In der zweiten Steuereinrichtung 15 werden die Ausgangssignale jedes der ADCs 13 aus jedem der vier Messkanäle ausgewertet und verarbeitet, wobei die Unterschiede in Phasenlage und Amplitude des zusammengesetzten Messsignals mit dem elektrischen Eingangssignal ein Maß z.B. für die Sauerstoffkonzentration im Farbstoff 8 sind.

Fig. 4 zeigt schematisch den Signalverlauf des elektrischen Eingangssignals V1, des elektrischen Messsignals V2 sowie beispielhaft für einen Messkanal das Ausgangssignal V3 des Schaltelements 7 und das Ausgangssignal V4 des Tiefpassfilters 9. Das elektrische Eingangssignal V1 ist ein Rechtecksignal mit einer Periode T, das elektrische Messsignal bzw. empfangene Lumineszenzsignal V2 ist sägezahnartig ausgebildet. Durch die Steuerung des Schaltelements 7 beschreibt das Ausgangssignal V3 des Schaltelements 7 nur einen Teilabschnitt des elektrischen Messsignals V2, in diesem Fall einen Teil der Anstiegsflanke, und das Ausgangssignal V4 des Tiefpassfilters stellt das integrierte Messsignal V3 dar.

Mit der erfindungsgemäßen Gegenstand wird eine Vorrichtung bzw. ein Verfahren zur Messung eines periodischen Signals bereitgestellt, die bzw. das eine relativ geringe Leistungsaufnahme und damit Eigenerwärmung aufweist, einfache und günstige Bauelemente enthält, eine effiziente und genaue Messung gewährleistet und besonders gut geeignet für den Einsatz in explosionsgefährdeten Umgebungen ist.

**Patentansprüche**

1. Sensor (1) für die Prozessmesstechnik zur Messung eines periodischen Signals mit
einer ersten Steuereinrichtung (3) zur Erzeugung eines periodischen elektrischen Eingangssignals (V1) der Periode T,
einer Lichtquelle (6) zur Erzeugung eines auf ein Messobjekt (8) gerichteten optischen Eingangssignals aus dem elektrischen Eingangssignal (V1) der Periode T,
einem optischen Empfänger (10) zur Erfassung und Umwandlung des von dem Messobjekt reflektierten Signals, das dem in Phase und Amplitude veränderten optischen Eingangssignal entspricht, in ein elektrisches Messsignal (V2) der Periode T, und
einer Mehrzahl von parallel zwischen dem optischen Empfänger (10) und einer zweiten Steuereinrichtung (15) geschalteten Messkanälen, die jeweils in Reihe geschaltet ein Schaltelement (7), ein Filterelement (9) und einen Analog-Digital-Wandler (13) aufweisen, wobei die zweite Steuereinrichtung (15) zur Auswertung der Messsignale von der Mehrzahl der Messkanäle geeignet ist,
wobei an der Mehrzahl von Messkanälen jeweils das elektrische Messsignal (V2) anliegt,
wobei die erste Steuereinrichtung (3) mit der Mehrzahl von Schaltelementen (7) verbunden und dazu eingerichtet ist, die Schaltelemente (7) für jeweils unterschiedliche Zeitintervalle derart anzuschalten, dass das elektrische Messsignal (V2) wiederholt in gleich langen, pro Messkanal disjunkten Zeitabschnitten abgegriffen wird, so dass sich die Zeitabschnitte in jedem der Messkanäle nicht überlappen, und
wobei die Analog-Digital-Wandler (13) eine maximale Abtastrate kleiner als $2 \times \dfrac{1}{T}$ aufweisen.

2. Sensor (1) nach Anspruch 1, **dadurch gekennzeichnet, dass** das elektrische Eingangssignal als Rechtecksignal ausgebildet ist.

3. Sensor (1) nach einem der vorhergehenden Ansprüche, **dadurch gekennzeichnet, dass** das Filterelement ein Tiefpass (9) ist.

4. Sensor (1) nach einem der vorhergehenden Ansprüche, **dadurch gekennzeichnet, dass** die Schaltelemente (7) in jedem der Mehrzahl von Messkanälen als CMOS-Schalter bzw. -Umschalter ausgebildet ist.

5. Sensor (1) nach einem der vorhergehenden Ansprüche, **dadurch gekennzeichnet, dass** die erste Steuereinrichtung (3) mit der zweiten Steuereinrichtung (15) verbunden und synchronisiert ist.

6. Sensor (1) nach einem der vorhergehenden Ansprüche, **dadurch gekennzeichnet, dass** ein integrierter Schaltkreis die erste Steuereinrichtung (3), die zweite Steuereinrichtung (15) sowie die Mehrzahl der Analog-Digital-Wandler (13) aufweist.

7. Sensor (1) nach einem der vorhergehenden Ansprüche, **dadurch gekennzeichnet, dass** die erste Steuereinrichtung (3) einen Pulsweitenmodulation-(PWM-) Generator aufweist.

8. Verfahren zur Messung eines periodischen Messsignals mit folgenden Schritten:

Senden eines optischen Eingangssignals einer Lichtquelle (6) auf Basis eines elektrischen Eingangssignals (V1) der Periode T auf ein Messobjekt (8),

Empfangen und Umwandeln eines optischen Messsignals, das dem in Phase und Amplitude veränderten optischen Eingangssignal entspricht, in einem optischen Empfänger (10) zu einem elektrischen Messsignal (V2) der Periode T,

Abgreifen des elektrischen Messsignals (V2) mit jedem einer Mehrzahl von parallel geschalteten Messkanälen, die jeweils in Reihe geschaltet ein Schaltelement (7), einen Tiefpass (9) und einen Analog-Digital-Wandler (13) aufweisen, wobei in jedem der Mehrzahl von Messkanälen das gleiche elektrische Messsignal (V2) verarbeitet wird, wobei das elektrische Messsignal (V2) in jedem der Mehrzahl von Messkanälen in jeweils disjunkten, periodisch wiederkehrenden Zeitintervallen derart abgegriffen wird, dass sich die Zeitabschnitte in jedem der Messkanäle nicht überlappen, wobei eine erste Steuereinrichtung (3) die Schaltelemente (7) in jedem der Mehrzahl von Messkanälen betätigt, wobei das elektrische Messsignal jedes Zeitintervalls in jedem der Mehrzahl von Messkanälen in dem Tiefpass (9) integriert, in dem Analog-Digital-Wandler (13) umgewandelt und in einer zweiten Steuereinrichtung (15) ausgewertet wird, und wobei in jedem Analog-Digital-Wandler (13) der Mehrzahl von Messkanälen die maximale Abtastrate kleiner als $2 \times \dfrac{1}{T}$ ist.

9. Verfahren nach Anspruch 8, **dadurch gekennzeichnet, dass** die Zeitintervalle in jedem der Mehrzahl von Messkanälen gleich lang sind.

10. Verfahren nach einem der Ansprüche 8 bis 9, **dadurch gekennzeichnet, dass** das elektrische Eingangssignal (V1) als Rechtecksignal ausgebildet ist.

11. Verfahren nach einem der Ansprüche 8 bis 10, **dadurch gekennzeichnet, dass** die erste Steuereinrichtung (3) und die zweite Steuereinrichtung (15) miteinander synchronisiert sind.

**Claims**

1. Sensor (1) for process measuring technology for measuring a periodic signal with
a first control device (3) for generating a periodic electrical input signal (V1) of period T,
a light source (6) for generating an optical input signal directed to an object (8) to be measured from the electrical input signal (V1) of period T,
an optical receiver (10) for detecting and converting the signal, which is reflected from the measurement object and corresponds to the optical input signal modified in phase and amplitude, into an electrical measurement signal (V2) of period T, and
a plurality of measuring channels which are switched in parallel between the optical receiver (10) and a second control device (15) and which comprise a switching element (7), a filter element (9) and an analog-digital converter (13) switched in series, wherein the second control device (15) is suitable for evaluating the measurement signals from the plurality of measuring channels,
wherein the electrical measurement signal (V2) contacts each of the plurality of measuring channels,
wherein the first control device (3) is connected to the plurality of switching elements (7) and is set up to switch on the switching elements (7) for the respective different time intervals in such a way that the electrical measurement signal (V2) is repeatedly picked up in equal length time sections disjoined per measuring channel, so that the time sections in each of the measuring channels do not overlap, and
wherein the analog-digital converters (13) have a maximum sampling rate lower than $2 \text{ x } \dfrac{1}{T}$.

2. Sensor (1) according to claim 1, **characterized in that** the electrical input signal is configured as a square wave signal.

3. Sensor (1) according to one of the preceding claims, **characterized in that** the filter element is a low pass filter (9).

4. Sensor (1) according to one of the preceding claims, **characterized in that** the switching element (7) in each of the plurality of measuring channels is configured as a CMOS switch or changeover switch.

5. Sensor (1) according to one of the preceding claims, **characterized in that** the first control device (3) is connected to and synchronized with the second control device (15).

6. Sensor (1) according to one of the preceding claims, **characterized in that** an integrated switching circuit comprises the first control device (3), the second control device (15) as well as the plurality of analog-digital converters (13).

7. Sensor (1) according to one of the preceding claims, **characterized in that** the first control device (3) has a pulse width modulation (PWM) generator.

8. Method for measuring a periodic measurement signal with the following steps:

sending an optical input signal of a light source (6) based on an electrical input signal (V1) of the period T to an object (8) to be measured, receiving and converting an optical measurement signal, which corresponds to the optical input signal which is modified in phase and amplitude, in an optical receiver (10) into an electrical measurement signal (V2) of period T, picking up the electrical measurement signal (V2) with each of a plurality of parallel-switched measuring channels which each have a switching element (7), a low pass filter (9) and an analog-digital converter (13) switched in series, wherein the same electrical measurement signal (V2) is processed in each of the plurality of measuring channels, wherein the electrical measurement signal (V2) is picked up in each of the plurality of measuring channels in respective disjoined periodically recurring time intervals in such a way that the time sections in each of the measuring channels do not overlap, wherein a first control device (3) actuates the switching elements (7) in each of the plurality of measuring channels, wherein the electrical measurement signal of each time interval in each of the plurality of measuring channels is integrated in the low pass filter (9), converted in the analog-digital converter (13) and evaluated in a second control device (15), and wherein in each analog-digital converter (13) of the plurality of measuring channels the maximum sampling rate is less than $2 \text{ x } \frac{1}{T}$.

9. Method according to claim 8, **characterized in that** the time intervals in each of the plurality of measuring channels are of the same length.

10. Method according to one of claims 8 to 9, **characterized in that** the electrical input signal (V1) is configured as a square wave signal.

11. Method according to one of claims 8 to 10, **characterized in that** the first control device (3) and the second control device (15) are synchronized with one another.

**Revendications**

1. Capteur (1) pour la technique de mesure de processus d'un signal périodique comprenant

un premier dispositif de commande (3) destiné à produire un signal d'entrée électrique (V1) périodique de la période T,

une source de lumière (6) destinée à produire un signal d'entrée optique dirigé sur un objet à mesurer (8) à partir du signal d'entrée électrique (V1) de la période T,

un récepteur optique (10) destiné à acquérir et à convertir le signal réfléchi par l'objet à mesurer, qui correspond au signal d'entrée optique modifié en phase et en amplitude, en signal de mesure électrique (V2) de la période T,

une pluralité de canaux de mesure montés en parallèle entre le récepteur optique (10) et un deuxième dispositif de commande (15), qui présentent, respectivement montés en série, un élément de commutation (7), un élément de filtre (9) et un convertisseur analogique-numérique (13), dans lequel le deuxième dispositif de commande (15) est adapté à évaluer les signaux de mesure de la pluralité des canaux de mesure,

dans lequel respectivement le signal de mesure électrique (V2) s'applique à la pluralité de canaux de mesure,

dans lequel le premier dispositif de commande (3) est relié à la pluralité d'éléments de commutation (7) et est conçu pour activer les éléments de commutation (7) pendant des intervalles de temps respectivement différents, de telle sorte que le signal de mesure électrique (V2) est détecté à plusieurs reprises par laps de temps disjoints de même longueur pour chaque canal de mesure, de sorte que les laps de temps dans chacun des canaux de mesure ne se chevauchent pas, et

dans lequel le convertisseur analogique-numérique (13) présente une fréquence d'échantillonnage maximale inférieure à $2 \times \frac{1}{T}$.

2. Capteur (1) selon la revendication 1, **caractérisé en ce que** le signal d'entrée électrique est réalisé sous la forme d'un signal rectangulaire.

3. Capteur (1) selon l'une quelconque des revendications précédentes, **caractérisé en ce que** l'élément de filtre est un filtre passe-bas (9).

4. Capteur (1) selon l'une quelconque des revendications précédentes, **caractérisé en ce que** les éléments de commutation (7) dans chaque canal de la pluralité de canaux de mesure sont réalisés sous la forme de commutateurs à semi-conducteur à oxyde de métal complémentaire.

5. Capteur (1) selon l'une quelconque des revendications précédentes, **caractérisé en ce que** le premier dispositif de commande (3) est relié au deuxième dispositif de commande (15) et synchronisé avec celui-ci.

6. Capteur (1) selon l'une quelconque des revendications précédentes, **caractérisé en ce qu'**un circuit intégré comprend le premier dispositif de commande (3), le deuxième dispositif de commande (15) ainsi que la pluralité des convertisseurs analogiques-numériques (13).

7. Capteur (1) selon l'une quelconque des revendications précédentes, **caractérisé en ce que** le premier dispositif de commande (3) présente un générateur de modulation d'impulsions en largeur.

8. Procédé de mesure d'un signal de mesure périodique, comprenant les étapes suivantes :

l'émission d'un signal d'entrée optique d'une source de lumière (6) sur la base d'un signal d'entrée électrique (V1) de la période T sur un objet à mesurer (8),
la réception et la conversion d'un signal de mesure optique, qui correspond au signal d'entrée optique modifié en phase et en amplitude, dans un récepteur optique (10), en signal de mesure électrique (V2) de la période T,
la détection du signal de mesure électrique (V2) avec chacun d'une pluralité de canaux de mesure montés en parallèle, qui présentent, respectivement montés en série, un élément de commutation (7), un filtre passe-bas (9) et un convertisseur analogique-numérique (13),
dans lequel le même signal de mesure électrique (V2) est traité dans chacun de la pluralité de canaux de mesure,
dans lequel le signal de mesure électrique (V2) est détecté par intervalles de temps récurrents, respectivement disjoints, dans chacun de la pluralité de canaux de mesure, de telle sorte que les laps de temps dans chacun des canaux de mesure ne se chevauchent pas, dans lequel un premier dispositif de commande (3) actionne les éléments de commutation (7) dans chacun de la pluralité de canaux de mesure,
dans lequel le signal de mesure électrique de chaque intervalle de temps dans chacun de la pluralité de canaux de mesure est intégré dans le filtre passe-bas (9), converti dans le convertisseur analogique-numérique (13) et évalué dans un deuxième dispositif de commande (15), et

dans lequel, dans chaque convertisseur analogique-numérique (13) de la pluralité de canaux de mesure, la vitesse d'échantillonnage maximale est inférieure à $2 \times \frac{1}{T}$.

9. Procédé selon la revendication 8, **caractérisé en ce que** les intervalles de temps dans chacun de la pluralité de canaux de mesure sont de même longueur.

10. Procédé selon l'une quelconque des revendications 8 à 9, **caractérisé en ce que** le signal d'entrée électrique (V1) est réalisé sous la forme d'un signal rectangulaire.

11. Procédé selon l'une quelconque des revendications 8 à 10, **caractérisé en ce que** le premier dispositif de commande (3) et le deuxième dispositif de commande (15) sont synchronisés l'un avec l'autre.

Fig. 1a

Fig. 1b

EP 2 936 091 B1

Fig. 2

EP 2 936 091 B1

Fig. 3

Fig. 4

EP 2 936 091 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8078246 B